# EUROPÄISCHE PATENTSCHRIFT

(11) **EP 0 582 825 B1**
(45) Veröffentlichungstag und Bekanntmachung des Hinweises auf die Patenterteilung: **21.04.1999**
(21) Anmeldenummer: 93110620.7
(22) Anmeldetag: 19.02.1990
(51) Int. Cl.: C07D 213/81

(54) **Amidierung von Pyridinen**
Amidation of pyridines
Amidation de pyridines

(30) Priorität: 28.02.1989 CH 736/89; 04.12.1989 CH 4323/89
(43) Veröffentlichungstag der Anmeldung: 16.02.1994
(62) Teilanmeldung aus: 90103106.2
(73) Patentinhaber: F. HOFFMANN-LA ROCHE AG, 4002 Basel (CH)
(72) Erfinder: Scalone, Michelangelo, Dr., CH-4127 Birsfelden (CH)
(74) Vertreter: Mahé, Jean

(56) Entgegenhaltungen:
- GB-A- 2 163 746
- US-A- 2 749 350
- CHEMICAL ABSTRACTS, Band 109, Nr. 25, 19. Dezember 1988, Columbus, Ohio, USA IMHOF, RENE et al. "N-(2-Aminoethyl)heterocycle- carboxamides, their prepara- tion, and their use for treatment of depression and parkinsonism" Seite 868, Zusammenfassung- -Nr. 231 063h
- CHEMICAL ABSTRACTS, Band 73, Nr. 25, 21. Dezember 1970, Columbus, Ohio, USA SHVARTSBERG, M.S. et al. "New reaction of acetylenes with terminal triple bonds" Seite 349, Zusammenfassung- -Nr. 130 851w

## Beschreibung

Die vorliegende Erfindung betrifft ein neues Verfahren zur Herstellung von Pyridin-2-carboxamiden der allgemeinen Formel 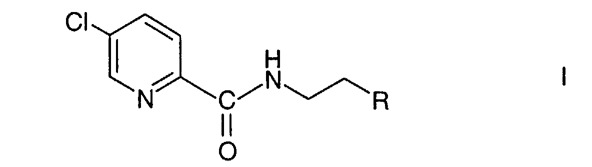 worin R Amino oder einen in Amino überführbaren Rest bedeutet,
und von pharmazeutisch verwendbaren Säureadditionssalzen derjenigen Verbindung, worin R Amino bedeutet.

Die Verbindungen der obigen Formel I, worin R einen in Amino überführbaren Rest bedeutet, sind wertvolle Zwischenprodukte und können beispielsweise zur Herstellung von N-(2-Aminoäthyl)-5-chlorpyridin-2-carboxamid, d.h. der Verbindung der Formel I, worin R amino bedeutet, verwendet werden. Diese Verbindung sowie deren pharmazeutisch verwendbare Säureadditionssalze besitzen eine ausgeprägte, reversible und hoch selektive Monoaminooxidase B (MAO-B) hemmende Eigenschaft und eignen sich demnach zur Behandlung von depressiven Zuständen, Parkinsonismus und kognitiven Erkrankungen. Verfahren zur Herstellung von N-(2-Aminoäthyl)-5-chlorpyridin-2-carboxamid und dessen pharmazeutisch verwendbaren Salzen-unter anderem aus Verbindungen der obigen Formel I, worin R einen in Amino überführbaren Rest bedeutet - sowie deren interessante pharmakologische Eigenschaften sind beispielsweise in der Britischen Patentschrift Nr. 2163746 beschrieben.

Der in der vorliegenden Beschreibung verwendete Ausdruck "nieder-Alkyl" betrifft geradkettige und verzweigte Kohlenwasserstoffreste mit 1-7, vorzugsweise 1-4, Kohlenstoffatomen, wie Methyl, Aethyl, Propyl, Isopropyl, Butyl, Isobutyl, sec.-Butyl, t-Butyl, Pentyl, Hexyl, Heptyl und dergleichen. In gleicher Weise betreffen die Ausdrücke "C₁₋₈-Alkyl" und C₁₋₄-Alkyl" entsprechende Kohlenwasserstoffreste mit 1-8 bzw. 1-4 Kohlenstoffatomen. Der Ausdruck "C₁₋₄-Alkoxy" bedeutet Alkyläthergruppe, worin "C₁₋₄-Alkyl" die obige Bedeutung hat. Der Ausdruck "Halogen" umfasst die vier Halogene Fluor, Chlor, Brom und Jod.

Das erfindungsgemässe Verfahren ist dadurch gekennzeichnet, dass man 2,5-Dichlorpyridin der Formel 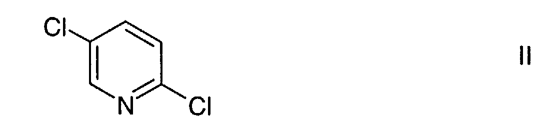 in Gegenwart eines Palladium-Phosphin-Katalysators
a) mit Kohlenmonoxid und einer Aminoverbindung der allgemeinen Formel 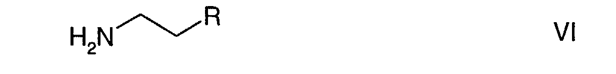 worin R die oben angegebene Bedeutung besitzt, umsetzt,
b) falls die Verbindung der Formel I erwünscht ist, worin R Amino bedeutet, nötigenfalls in einer erhaltenen Verbindung der Formel I, worin R einen in Amino überführbaren Rest bedeutet, diesen Rest in Amino überführt, und
c) erwünschtenfalls, die erhaltene Verbindung in ein pharmazeutisch verwendbares Säureadditionssalz überführt.

Die Amidierung von 2,5-Dichlorpyridin der Formel II mit Kohlenmonoxid und einer Aminoverbindung der Formel VI in Gegenwart eines Palladium-Phosphin-Katalysators kann man in an sich bekannter Weise durchführen. So kann man die Reaktion z.B. unter wasser- und sauerstofffreien Bedingungen in Gegenwart einer Base in einem unter den Reaktionsbedingungen inerten Lösungsmittel oder Lösungsmittelgemisch bei einer Temperatur zwischen etwa 60° und 150°C, vorzugsweise zwischen etwa 100° und 130°C, durchführen. Geeignete Basen sind organische Basen, wie tertiäre Amine, z.B. Trialkylamine, wie Trimethylamin, Triäthylamin, Aethyldiisopropylamin und dergleichen, Dialkylarylamine, wie N,N-Dimethylanilin und dergleichen, Triarylamine, wie Triphenylamin, Tritolylamin und dergleichen und anorganische Basen, wie Natriumbicarbonat, Kaliumbicarbonat, Calciumcarbonat und dergleichen, wobei auch überschüssiges Amin der Formel VI als Base dienen kann. Wird die Reaktion in Gegenwart einer anorganischen Base durchgeführt, kann die Verwendung eines Phasentransferkatalysators oder eines Kronenäthers von Vorteil sein. Die bevorzugte Base ist das Triäthylamin. Als Lösungsmittel kommen aliphatische und aromatische Kohlenwasserstoffe, wie Hexan, Benzol, Toluol, Xylol und dergleichen, halogenierte aliphatische und aromatische Kohlenwasserstoffe, wie Methylenchlorid, Chlorbenzol und dergleichen, Aether, wie Diäthyläther, Tetrahydrofuran, Dioxan und dergleichen, Ketone, wie Aceton, Methylpropylketon und dergleichen, Carbonsäureester, wie Methylacetat, Aethylacetat und dergleichen, Alkohole, wie t-Butanol und dergleichen, Dimethylformamid, Dimethylsulfoxid und dergleichen, in Frage. Bei der erfindungsgemässen Umsetzung ist der Druck nicht kritisch, weshalb man bei Atmosphärendruck oder erhöhten Drucken arbeiten kann, vorzugsweise bei etwa 10⁶ Pa.

Beim Katalysator handelt es sich um eine Palladium-Phosphin-Komplexverbindung, welche zweckmässigerweise in situ aus einer Palladiumkomponente und einem Phosphinliganden gebildet wird. Als Palladiumkomponente kommen metallisches Palladium, das gegebenenfalls auf ein Trägermaterial, wie Kohle, aufgetragen ist, oder ein Komplex bzw. ein Salz des 0-, 2- oder 4-wertigen Palladiums, wie Palladium-bis(dibenzylidenaceton), Palladiumchlorid, Palladiumacetat und dergleichen, in Frage. Die Menge an Palladiumkomponente beträgt zweckmässigerweise 0,0001-0,5 Mol %, vorzugsweise 0,01-0,1 Mol %. Als Phosphinligand kommen chirale und nicht chirale Mono- und Diphosphorverbindungen wie sie in Houben-Weyl, Methoden der organischen Chemie, Band El, Seite 106ff, Georg Thieme Verlag Stuttgart, 1982, und Aspects Homog. Catal., 4, 145-202 (1981) beschrieben sind, insbesondere solche der allgemeinen Formeln

P(R⁴)(R⁵)(R⁶) und (R⁴)(R⁵)P-(X)-P(R⁴)(R⁵)

worin R⁴, R⁵ und R⁶ die oben angegebene Bedeutung besitzen und X Binaphthyl oder eine der Gruppen -(CH₂)ₙ-, -CH₂CH₂-P(C₆H₅)-CH₂CH₂-, 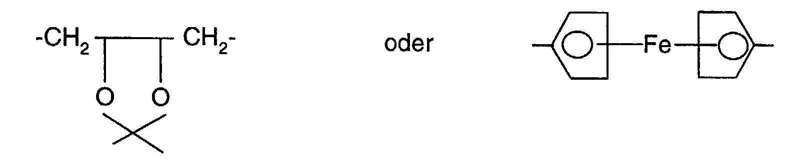 und n eine Zahl von 1-8 bedeuten,
in Frage.

Die Menge an Phosphinligand beträgt zweckmässigerweise 0,01-100 Mol pro Mol Palladium, vorzugsweise 0,1-10 Mol pro Mol Palladium. Zur in situ Herstellung der Palladium-Phosphin-Komplexverbindung verwendet man vorzugsweise Palladium-(II)-chlorid oder Palladium-(II)-acetat, Palladiumdichlor-bis(acetonitril) und Bis(diphenylphosphino)alkane, vorzugsweise 1,3-Bis(diphenylphosphino)propan.

Die Ueberführung von Verbindungen der Formel I, worin R einen in Amino überführbaren Rest bedeutet, in die Verbindung der Formel I, worin R Amino bedeutet, sowie die Ueberführung der letztgenannten Verbindung in pharmazeutisch verwendbare Säureadditionssalze ist beispielsweise in der Britischen Patentschrift Nr. 2163746 vorbeschrieben.

Das als Ausgangsmaterial verwendete 2,5-Dichlorpyridin sowie die als Ausgangsstoffe verwendeten Verbindungen der Formeln III und VI sind bekannt oder können in Analogie zu den bekannten Verbindungen hergestellt werden.

Die nachfolgenden Beispiele dienen der näheren Erläuterung der vorliegenden Erfindung; alle Temperaturen sind in Celsiusgraden angegeben.

### Beispiel 1

Ein 250 ml Autoklav wird mit 50 ml Toluol, 5,2 ml (37,2 mMol) Triäthylamin, 1,2 mg (0,0067 mMol) Palladiumchlorid, 5,6 mg (0,0135 mMol) 1,3-Bis-(diphenylphosphino)propan, 6,5 g (40,56 mMol) t-Butyl (2-aminoäthyl)-carbamat und 5,0 g (33,8 mMol) 2,5-Dichlorpyridin unter einer Argonatmosphäre beladen. Nach mehrmaligem Evakuieren und Aufdrücken mit 10⁶ Pa Stickstoff wird der Autoklav mit 10⁶ Pa Kohlenmonoxid begast und unter Rühren auf 130° erhitzt. Nach 24-stündigem Erhitzen auf 130° wird der Autoklav abgekühlt, geöffnet und entladen, und die orange-braune Suspension mit einem 1:1-Gemisch von Toluol und Aether als Eluierungsmittel durch 50 g Kieselgel filtriert. Die das erwünschte Produkt enthaltenden Fraktionen (dünnschichtchromatographisch bestimmt) werden vereinigt, auf ca.150 ml eingedampft und zur Abtrennung des Palladiums mit einer Lösung von 2 mg Natriumcyanid in 25 ml Wasser bei 70° über Nacht kräftig gerührt. Danach wird die organische Phase abgetrennt, mit Wasser gewaschen und eingedampft. Kristallisation des festen Rückstands aus Toluol/Hexan liefert 8,51 g (84%) t-Butyl [2-(5-chlorpyridin-2-carboxamido)äthyl]carbamat als weisse Kristalle, Smp. 106-108°.

### Beispiel 2

In zu Beispiel 1 analoger Weise wird die Amidierung von 5 g (33,8 mMol) 2,5-Dichlorpyridin in Gegenwart von 475 mg Palladium-dichlor-bis(triphenylphosphin) und 355 mg Triphenylphosphin durchgeführt. Aufarbeiten des Rohprodukts wie in Beispiel 1 beschrieben liefert 8,83 g (87,2%) reines t-Butyl [2-(5-chlorpyridin-2-carboxamido)äthyl]carbamat, das mit dem in Beispiel 1 erhaltenen Produkt identisch ist.

### Beispiel 3

In zu Beispiel 1 analoger Weise wird die Amidierung von 5 g (33,8 mMol) 2,5-Dichlorpyridin in Gegenwart von 2,0 mg Palladiumchlorid und 4,8 mg 1,3-Bis(diphenylphosphino)propan bei 110° durchgeführt. Aufarbeiten des Rohprodukts wie in Beispiel 1 beschrieben liefert 9,45 g (93,3%) reines t-Butyl [2-(5-chlorpyridin-2-carboxamido)äthyl]carbamat, das mit dem in Beispiel 1 erhaltenen Produkt identisch ist.

### Beispiel 4

In zu Beispiel 1 analoger Weise wird die Amidierung von 5 g (33,8 mMol) 2,5-Dichlorpyridin in Gegenwart von 11,4 mg Palladiumacetat und 75 mg 1,3-Bis(diphenylphosphino)propan durchgeführt. Aufarbeiten des Rohprodukts wie in Beispiel 1 beschrieben liefert 9,66 g (95,4%) reines t-Butyl [2-(5-chlorpyridin-2-carboxamido)äthyl]carbamat, das mit dem in Beispiel 1 erhaltenen Produkt identisch ist.

### Beispiel 5

In zu Beispiel 1 analoger Weise wird die Amidierung von 5 g (33,8 mMol) 2,5-Dichlorpyridin in Anwesenheit von 27 mg Bis(Palladium)-tris(dibenzylidenaceton)chloroformaddukt und 75 mg 1,3-Bis(diphenylphosphino)propan durchgeführt. Aufarbeiten des Rohprodukts wie in Beispiel 1 beschrieben liefert 9,20 g (90,8%) reines t-Butyl [2-(5-chlorpyridin-2-carboxamido)äthyl]-carbamat, das mit dem in Beispiel 1 erhaltenen Produkt identisch ist.

### Beispiel 6

In einem Rundkolben werden 1,0 g (6,75 mMol) 2,5-Dichlorpyridin mit 2,49 g (15,54 mMol) t-Butyl (2-aminoäthyl)carbamat, 197 mg Palladium-bis-(dibenzylidenaceton) und 147 mg 1,3-Bis(diphenylphosphino)propan in 10 ml Toluol bei 120° unter einer Kohlenmonoxidatmosphäre 2,7 Stunden zur Reaktion gebracht. Zu Beispiel 1 analog durchgeführtes Aufarbeiten des Rohprodukts liefert 1,7 g (83%) reines t-Butyl [2-(5-chlorpyridin-2-carboxamido)äthyl]carbamat, das mit dem in Beispiel 1 erhaltenen Produkt identisch ist.

### Beispiel 7

In zu Beispiel 1 analoger Weise wird die Amidierung von 5 g (33,8 mMol) 2,5-Dichlorpyridin in Gegenwart von 13,5 mg Palladium-dichlor-bis(acetonitril) und 80 mg 1,3-Bis(diphenylphosphino)propan in 50 ml Tetrahydrofuran durchgeführt. Aufarbeiten des Rohprodukts wie in Beispiel 1 beschrieben liefert 9,15 g (90,3%) reines t-Butyl [2-(5-chlorpyridin-2-carboxamido)äthyl]carbamat, das mit dem in Beispiel 1 erhaltenen Produkt identisch ist.

### Beispiel 8

In zu Beispiel 1 analoger Weise wird die Amidierung von 5 g (33,8 mMol) 2,5-Dichlorpyridin in Gegenwart von 13,5 mg Palladium-dichlor-bis(acetonitril) und 80 mg 1,3-Bis(diphenylphosphino)propan in 50 ml Aethylacetat durchgeführt. Aufarbeiten des Rohprodukts wie in Beispiel 1 beschrieben liefert 9,51 g (93,9%) reines t-Butyl [2-(5-chlorpyridin-2-carboxamido)äthyl]-carbamat, das mit dem in Beispiel 1 erhaltenen Produkt identisch ist.

### Beispiel 9

In zu Beispiel 2 analoger Weise wird die Amidierung von 5 g (33,8 mMol) 2,5-Dichlorpyridin in Gegenwart von 137 mg Tetrabutylammoniumjodid und 3,12 g Natriumbicarbonat durchgeführt. Aufarbeiten des Rohprodukts wie in Beispiel 1 beschrieben liefert 7,53 g (74,3%) reines t-Butyl [2-(5-chlorpyridin-2-carboxamido)äthyl]carbamat, das mit dem in Beispiel 1 erhaltenen Produkt identisch ist.

### Beispiel 10

In zu Beispiel 1 analoger Weise wird die Amidierung von 5 g (33,8 mMol) 2,5-Dichlorpyridin in Gegenwart von 12,1 g (202 mMol) Aethylendiamin, 8,8 mg Palladium-dichlor-bis(acetonitril) und 21 mg 1,3-Bis(diphenylphosphino)-propan bei 110° durchgeführt. Nach Zugabe von 1,91 g (35,5 mMol) Natriummethylat und Eindampfen des Reaktionsgemisches werden durch Ausschütteln mit 3N Salzsäure und anschliessendem Basischstellen der sauren wässrigen Lösung 5,05 g (75%) N-(2-Aminoäthyl)-5-chlorpyridin-2-carboxamid erhalten; Gehalt nach HPLC: 98,2 Gew.%, Gehalt an N,N'-Aethylen-bis(5-chlor-2-pyridincarboxamid): <1% (GC). Ueberführung der freien Base mit Chlorwasserstoff in Methanol ins Hydrochlorid und Kristallisation aus Methanol/Aether liefert N-(2-Aminoäthyl)-5-chlorpyridin-2-carboxamidhydrochlorid als weisse Kristalle, Smp. 191-195°.

### Beispiel 11

In zu Beispiel 1 analoger Weise wird die Amidierung von 10 g (67,6 mMol) 2,5-Dichlorpyridin in Gegenwart von 13,2 g (87,8 mMol) N-Benzyläthylendiamin, 11,5 g Palladiumchlorid und 26,5 mg 1,3-Bis(diphenylphosphino)propan bei 110° durchgeführt. Durch Ausschütteln des Reaktionsgemisches mit 1N Salzsäure und anschliessendem Basischstellen der sauren wässrigen Lösung wird N-[2-(Benzylamino)äthyl] -5-chlorpyridin-2-carboxamid als leicht gelbes Oel erhalten, welches durch Rühren in Hexan 16,61 g (85%) reines N-[2-(Benzylamino)äthyl]-5-chlorpyridin-2-carboxamid als leicht gelbe Kristalle liefert, Smp. 45-46°.

### Beispiel 12

In zu Beispiel 1 analoger Weise wird die Carbonylierung von 5,0 g (33,8 mMol) )2,5-Dichlorpyridin in 50 ml Methanol/Triäthylamin 1:1 in Gegenwart von 0,47 g Palladium-dichlor-bis(triphenylphosphin) bei 110° durchgeführt. Aufarbeiten des Rohproduktes wie in Beispiel 1 liefert 2,86 g (49%) rohen 5-Chlorpyridin-2-carbonsäuremethylester, welcher durch Kristallisation aus Hexan reinen 5-Chlorpyridin-2-carbonsäuremethylester in Form weisser Kristalle liefert, Smp. 83-86°.

## Patentansprüche

1. Verfahren zur Herstellung von Pyridin-2-carboxamiden der allgemeinen Formel 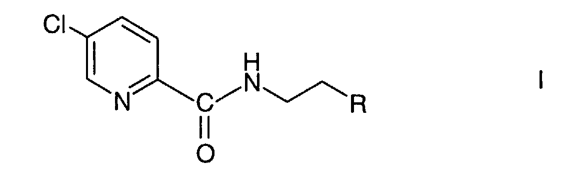 worin R Amino oder einen in Amino überführbaren Rest bedeutet,
und von pharmazeutisch verwendbaren Säureadditionssalzen derjenigen Verbindung, worin R Amino bedeutet, dadurch gekennzeichnet, dass man 2,5-Dichlorpyridin der Formel 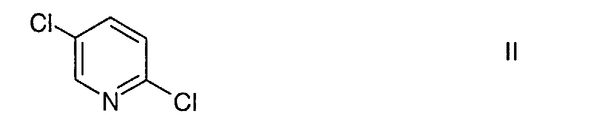 in Gegenwart eines Palladium-Phosphin-Katalysators
a) mit Kohlenmonoxid und einer Aminoverbindung der allgemeinen Formel 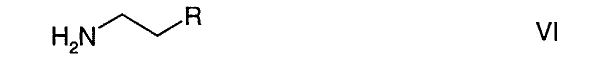 worin R die oben angegebene Bedeutung besitzt, umsetzt,
b) falls die Verbindung der Formel I erwünscht ist, worin R Amino bedeutet, nötigenfalls in einer erhaltenen Verbindung der Formel I, worin R einen in Amino überführbaren Rest bedeutet, diesen Rest in Amino überführt, und
c) erwünschtenfalls, die erhaltene Verbindung in ein pharmazeutisch verwendbares Säureadditionssalz überführt.

2. Verfahren nach Anspruch 1, dadurch gekennzeichnet, dass man die Reaktion in Gegenwart einer Base durchführt.

3. Verfahren nach Anspruch 2, dadurch gekennzeichnet, dass man als Base in Verfahrensstufe a) Triäthylamin verwendet.

4. Verfahren nach einem der Ansprüche 1-3, dadurch gekennzeichnet, dass man die Umsetzung gemäss Verfahrensstufe a) in einem aromatischen Kohlenwasserstoff, einem Aether oder einem Carbonsäureester durchführt.

5. Verfahren nach Anspruch 4, dadurch gekennzeichnet, dass man die Umsetzung in Toluol, Tetrahydrofuran oder Aethylacetat durchführt.

6. Verfahren nach einem der Ansprüche 1-5, dadurch gekennzeichnet, dass man die Umsetzung gemäss Verfahrensstufe a) bei einer Temperatur zwischen etwa 60° und 150°C durchführt.

7. Verfahren nach Anspruch 6, dadurch gekennzeichnet, dass man die Umsetzung bei einer Temperatur zwischen etwa 100° und 130°C durchführt.

8. Verfahren nach einem der Ansprüche 1-7, dadurch gekennzeichnet, dass man die Umsetzungen bei einem Druck von etwa 10⁵ bis etwa 10⁷ Pa durchführt.

9. Verfahren nach Anspruch 8, dadurch gekennzeichnet, dass man die Umsetzung gemäss Verfahrensstufe a) bei einem Druck von etwa 10⁶ Pa durchführt.

10. Verfahren nach einem der Ansprüche 2-9, dadurch gekennzeichnet, dass der Palladium-Phosphin-Katalysator in situ hergestellt wird.

11. Verfahren nach Anspruch 10, dadurch gekennzeichnet, dass der Palladium-Phosphin-Katalysator aus 0,0001-0,5 Mol % metallischem Palladium oder einem Komplex bzw. Salz des 0-, 2- oder 4-wertigen Palladiums und 0,01-100 Mol pro Mol Palladium einer Phosphorverbindung der allgemeinen Formeln
P(R⁴)(R⁵)(R⁶) und (R⁴)(R⁵)P-(X)-P(R⁴)(R⁵)
worin R⁴, R⁵ und R⁶ unabhängig voneinander je C₁₋₈-Alkyl, Cyclohexyl, Benzyl, Phenyl oder durch C₁₋₄-Alkyl, C₁₋₄-Alkoxy, Halogen, Trifluormethyl oder Phenyl substituiertes Phenyl und X Binaphthyl oder eine der Gruppen -(CH₂)ₙ-, -CH₂CH₂-P(C₆H₅)-CH₂CH₂-, 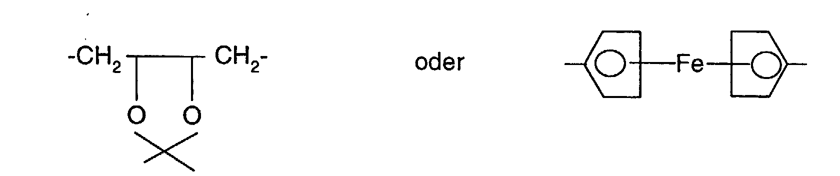 und n eine Zahl von 1-8 bedeuten,
hergestellt werden.

12. Verfahren nach Anspruch 11, dadurch gekennzeichnet, dass man Palladium-(II)-chlorid oder Palladium-(II)-acetat und ein Bis(diphenylphosphino)alkan, vorzugsweise 1,3-Bis(diphenylphosphino)propan, zur Herstellung des Palladium-Phosphin-Katalysators verwendet.

13. Verfahren nach Anspruch 11 oder 12, dadurch gekennzeichnet, dass man 0,01-0,1% Palladiumkomponente und 0,1-10 Mol Phosphinligand pro Mol Palladium verwendet.

14. Verfahren nach einem der Ansprüche 1-13, dadurch gekennzeichnet, dass man als Aminoverbindung der Formel VI Aethylendiamin oder t-Butyl (2-aminoäthyl)carbamat verwendet.

## Claims

1. A process for the manufacture of pyridine-2-carboxamides of the general formula 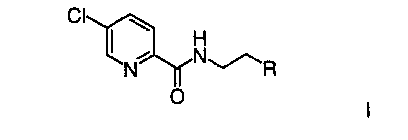 wherein R signifies amino or a residue convertible into amino,
and of pharmaceutically usable acid addition salts of that compound, in which R signifies amino, which process comprises reacting 2,5-dichloropyridine of the formula 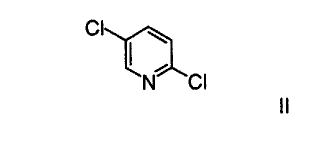 in the presence of a palladium-phosphine catalyst
a) with carbon monoxide and an amino compound of formula 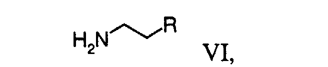 wherein R has the significance given above,
b) where the compound of formula I, in which R signifies amino is desired, if necessary converting into amino the residue R in a resulting compound of formula I in which R signifies a residue convertible into amino, and
c) if desired, converting the resulting compound into a pharmaceutically usable acid addition salt.

2. A process according to claim 1, wherein the reaction is carried out in the presence of a base.

3. A process according to claim 2, wherein triethylamine is used as the base in process variant a).

4. A process according to any one of claims 1 - 3, wherein the reaction in accordance with process variant a) is carried out in an aromatic hydrocarbon, an ether or a carboxilic acid ester.

5. A process according to claim 4, wherein the reaction is carried out in toluene, tetrahydrofuran or ethyl acetate.

6. A process according to any one of claims 1 - 5, wherein the reaction is carried out at a temperature between about 60° and 150°.

7. A process according to claim 6, wherein the reaction is carried out at a temperature between about 100° and 130°.

8. A process according to any one of claims 1 - 7, wherein the reactions are carried out at a pressure of about 10⁵ to about 10⁷ Pa.

9. A process according to claim 8, wherein the process variant a) is carried out at a pressure of about 10⁶ Pa.

10. A process according to any one of claims 2 - 9, wherein the palladium-phosphine catalist is prepared in situ.

11. A process according to claim 10, wherein the palladium-phosphine catalyst is prepared from 0.0001 - 0.5 mol% of metallic palladium or a complex or salts of 0-, 2- or 4-valent palladium and 0.01 - 100 mol per mol of palladium of a phosphorus compound of the general formulae
P(R⁴)(R⁵)(R⁶) and (R⁴)(R⁵)P-(x)-P(R⁴)(R⁵)
wherein R⁴, R⁵ and R⁶ each independently signify C₁₋₈-alkyl, cyclohexyl, benzyl, phenyl or phenyl, which is substituted by C₁₋₄-alkyl, C₁₋₄alkoxy, halogen, trifluoromethyl or phenyl and X signifies binaphthyl or one of the groups -(CH₂)ₙ-, -CH₂CH₂-P(C₆H₅)-CH₂-CH₂-, 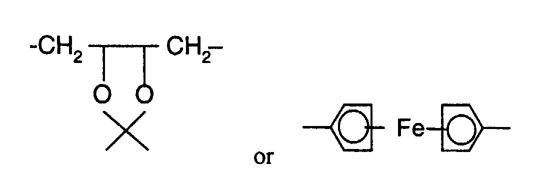 and n signifies a number of 1 - 8.

12. A process according to claim 11, wherein palladium-(II) chloride or palladium-(II) acetate and a bis(diphenylphosphino)alkane, preferably 1,3-bis-(diphenylphosphino)propane, is used for the preparation of the palladium-phosphine catalyst.

13. A process according to claims 11 and 12, wherein 0.01 - 0.1% of palladium component and 0.1 - 10 mol of phosphine ligand are used per mol of palladium.

14. A process according to any one of claims 1-13, wherein ethylenediamine or t-butyl (2-aminoethyl)carbamate is used as the amino compound of formula VI.

## Revendications

1. Procédé pour la préparation de pyridine-2-carboxamides de formule générale : 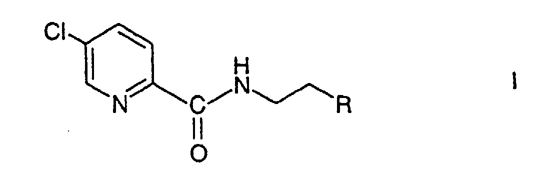 dans laquelle R représente un groupe amino ou un groupe convertible en un groupe amino, et des sels d'addition acides acceptables pour l'usage pharmaceutique du composé pour lequel R représente un groupe amino, caractérisé en ce que l'on fait réagir la 2,5-dichloropyridine de formule 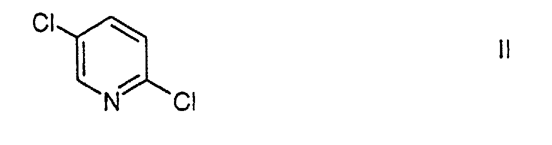 en présence d'un catalyseur du type phosphine-palladium
a) avec le monoxyde de carbone et un dérivé aminé de formule générale 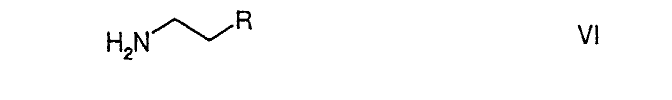 dans laquelle R a les significations indiquées ci-dessus,
b) et si on désire préparer le composé de formule I dans laquelle R représente un groupe amino, partant d'un composé de formule I dans laquelle R représente un groupe convertible en un groupe amino, on convertit ce groupe en le groupe amino, et
c) si on le désire, on convertit le composé obtenu en un sel d'addition acide acceptable pour l'usage pharmaceutique.

2. Procédé selon la revendication 1, caractérisé en ce que la réaction est réalisée en présence d'une base.

3. Procédé selon la revendication 2, caractérisé en ce que la base utilisée au stade opératoire a) est la triéthylamine.

4. Procédé selon l'une des revendications 1 à 3, caractérisé en ce que la réaction du stade opératoire a) est réalisée dans un hydrocarbure aromatique, un éther ou un ester d'acide carboxylique.

5. Procédé selon la revendication 4, caractérisé en ce que la réaction est réalisée dans le toluène, le tétrahydrofuranne ou l'acétate d'éthyle.

6. Procédé selon l'une des revendications 1 à 5, caractérisé en ce que la réaction du stade opératoire a) est réalisée à une température allant d'environ 60 à 150°C.

7. Procédé selon la revendication 6, caractérisé en ce que la réaction est réalisée à une température allant d'environ 100 à 130°C.

8. Procédé selon l'une des revendications 1 à 7, caractérisé en ce que les réactions sont réalisées sous une pression d'environ 10⁵ à 10⁷ Pa.

9. Procédé selon revendication 8, caractérisé en ce que la réaction du stade opératoire a) est réalisée sous une pression d'environ 10⁶ Pa.

10. Procédé selon l'une des revendications 2 à 9, caractérisé en ce que le catalyseur du type phosphine-palladium est préparé in situ.

11. Procédé selon la revendication 10, caractérisé en ce que le catalyseur du type phosphine-palladium est préparé à partir de 0,0001 à 0,5 mol % de palladium métallique ou d'un complexe ou sel du palladium de valence 0, 2 ou 4, et de 0,01 à 100 mol, pour 1 mol de palladium, d'un composé du phosphore répondant à l'une des formules générales
P(R⁴)(R⁵)(R⁶) et (R⁴)(R⁵)P-(X)-P(R⁴)(R⁵)
dans lesquelles R⁴, R⁵ et R⁶ représentent chacun, indépendamment les uns des autres, un groupe alkyle en C1-C8, cyclohexyle, benzyle, phényle ou phényle substitué par des groupes alkyle en C₁-C₄, alcoxy en C₁-C₄, des halogène, trifluorométhyle ou phényle, et X représente un groupe binaphtyle ou l'un des groupes -(CH₂)ₙ, -CH₂CH₂-P(C₆H₅)-CH₂CH₂- 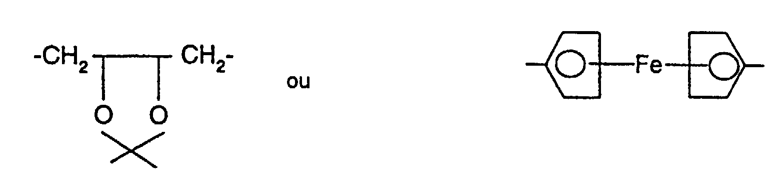 et n est un nombre allant de 1 à 8.

12. Procédé selon la revendication 11, caractérisé en ce que, pour la préparation du catalyseur du type phosphine-palladium, on utilise le chlorure de palladium-II ou l'acétate de palladium-II et un bis-(diphénylphosphino)-alcane, de préférence le 1,3-bis-(diphénylphosphino)-propane.

13. Procédé selon la revendication 11 ou 12, caractérisé en ce que l'on utilise de 0,01 à 0,1 % du composant du palladium et de 0,1 à 10 mole du ligand consistant en une phosphine par mole de palladium.

14. Procédé selon l'une des revendications 1 à 13, caractérisé en ce que le dérivé aminé de formule VI est l'éthylènediamine ou le (2-aminoéthyl)-carbamate de tert-butyle.
